# EUROPEAN PATENT SPECIFICATION

(11) **EP 0 722 700 B1**
(45) Date of publication and mention of the grant of the patent: **16.12.1998**
(21) Application number: 95830103.8
(22) Date of filing: 17.03.1995
(51) Int. Cl.: A61F 2/06

(54) **A coronary endoprosthesis and a method for its fabrication**
Koronare Endoprothese und ihr Herstellungsverfahren
Endoprothèse coronaire et sa méthode de fabrication

(30) Priority: 20.01.1995 IT BO950015
(43) Date of publication of application: 24.07.1996
(73) Proprietor: X-TRODE S.r.l., 40129 Bologna (IT)
(72) Inventor: Borghi, Enzo, I-40054 Budrio (Bologna) (IT)
(74) Representative: Lanzoni, Luciano

(56) References cited:
- WO-A-83/00997
- WO-A-93/13825
- WO-A-94/20044
- US-A- 4 800 882

## Description

The present invention relates to a coronary endoprosthesis and to a method for its fabrication.

The art field of surgery currently embraces various types of coronary endoprostheses, referred to more briefly as "stents" by those skilled in the art, designed for use in cases where a patient may be diagnosed as suffering from a vascular occlusion, in particular a blockage of the coronary arteries which have the important function of carrying blood to the cardiac muscle.

A stent consists generally in a tubular element of varying thickness and geometry that serves, when introduced by means of a catheter and expanded with an inflatable balloon, to establish a small duct matching the contours of the vessel into which it is inserted; the purpose of the endoprosthesis is therefore to keep the blood vessel open.

The art field of biomedical engineering embraces a variety of endoprosthetic solutions: of particular interest in the light of the present specification are those typified, for example as in the case of US Patent 4,800,882, which discloses the closest prior art by the fabrication of a stent from a single metal wire formed initially into a serpentine appearing as a plurality of flat loops with rectilinear members and interconnecting bends.

The flat serpentine is then deformed mechanically in such a way that the single loops are rendered substantially cylindrical in section and combine in alternation with those preceding or following in sequence to create a tubular cylindrical section centred on a longitudinal axis.

A stent structured in this manner is compressed radially over a balloon catheter and inserted into the coronary vessel to the point of penetrating the occluded zone, then spread radially and plastically by expanding the balloon, though without undergoing any axial deformation, so as to re-establish the natural dimensions of the lumen.

The main drawbacks betrayed by such a solution stem most significantly from an insufficient degree of resistance to radial deformation (one of the prime requirements in a coronary endoprosthesis), which is attributable to the particular type of structure utilizing a single slender wire; the problem arises from the fact that the coronary artery is a vessel through which blood flows under pressure, and the implanted stent must therefore withstand a notable pressor action. In addition, there are problems of constructional "rigidity" in this type of solution due to the fact that the structure of the stent (the longitudinal dimension of which is standard and invariably fixed) does not allow a surgeon to obtain the configuration best able to deal with the pathological situation encountered in each patient: in short, the surgeon has no way of modifying the dimensions of the prosthesis preoperatively to suit the pathological attributes of the case in hand, but is obliged to effect an "empirical" adaptation of the stent in some way or even to replace it with another, thus prolonging the operation.

Another solution is illustrated in European Patent EP 335 341. This discloses a stent appearing as a plurality of cylindrical elements fashioned from a material of suitably thin gauge, which exhibits a series of through slots extending parallel with the longitudinal axis of the prosthetic structure; the single cylindrical elements are interconnected by way of flexible bridging elements that consist in deformable extensions of the cylindrical elements themselves. Again, the stent is implanted in the blood vessel utilizing a balloon catheter so that the necessary radial deformation can be induced in the cylindrical elements.

In this solution too, however, there are drawbacks deriving from the type of structure adopted for the stent, which is one exhibiting extensive metallic surface areas fragmented by relative notches and grooves; over time, these can create interstices favouring the build-up of arteriosclerotic plaque, of which the physical impact is not inconsiderable.

There is also a lack of operational flexibility in respect of the different pathological situations encountered from patient to patient, since it is all but totally impossible to diversify the axial configuration of the stent unless by dividing the structure into a plurality of discrete parts: this contingency occurs frequently when there are at least two occlusions of the same coronary artery separated by a distance more than the axial length of the stent. A further "anomalous" situation is that which can occur when the site of the occlusion in a coronary artery is near to a junction between that artery and another afferent blood vessel: in effect, the implantation of this stent would cause a partial closure of the latter vessel.

The object of the present invention, accordingly, is to overcome the drawbacks mentioned above by providing a coronary endoprosthesis structured in such a way as to afford an advantageous balance in terms of the ratio between the quantity of material utilized and the corresponding empty spaces, also a significant facility for varying the configuration of the prosthetic structure in order to accommodate the particular pathological situations encountered from patient to patient, while maintaining a high specification as regards mechanical strength, and physical and chemical compatibility with the blood vessel and the bloodstream. The present invention is specified in claim 1. Methods of fabrication an endoprosthesis are specified in claims 14-17.

The invention will now be described in detail, by way of example, with the aid of the accompanying drawings, in which:
- fig 1 shows the coronary endoprosthesis according to the invention in a contracted, non-operative configuration, viewed in side elevation;
- fig 2 shows the coronary endoprosthesis of fig 1 in an expanded, operative configuration, viewed in a further side elevation;
- fig 2a shows an alternative embodiment of the endoprosthesis illustrated in figs 1 and 2, viewed in a side elevation;
- figs 3a-b-c-d illustrate corresponding steps in a first method of fabricating discrete modules making up the endoprosthesis disclosed, of which fig 3a is a perspective view and the remaining figures are all elevations;
- figs 4a-b-c illustrate corresponding steps in a second method of fabricating discrete modules making up the endoprosthesis disclosed, of which fig 4a is a perspective view and the remaining figures are elevations;
- fig 5 illustrates one step in a third method of fabricating modules making up the endoprosthesis disclosed, viewed in perspective;
- fig 6 is a perspective view of a module fabricated by the methods illustrated in the previous drawings and suitable for use in the solution of fig 2a;
- fig 7 illustrates a further step of deforming the module of fig 6 to obtain the configuration for an endoprosthesis as in figs 1 and 2;
- figs 8, 9 and 10 illustrate respective steps in the procedure of introducing and implanting the endoprosthesis of the preceding drawings, all seen in side elevation;
- fig 11 shows a particular situation in which the endoprosthesis disclosed might be implanted, viewed in a side elevation;
- figs 12 to 16 illustrate respective steps in a fourth method of fabricating the endoprosthesis according to the invention, of which figs 12 and 13 are perspectives, figs 14 and 15 are elevations and fig 16 is a further perspective;
- figs 17 and 18 illustrate two possible radioscope observations of the endoprosthesis fabricated by the method of figs 12 to 16, both of which viewed in plan, during the respective steps of positioning and expanding the endoprosthesis;
- fig 19 is the section through XIX-XIX in fig 18.

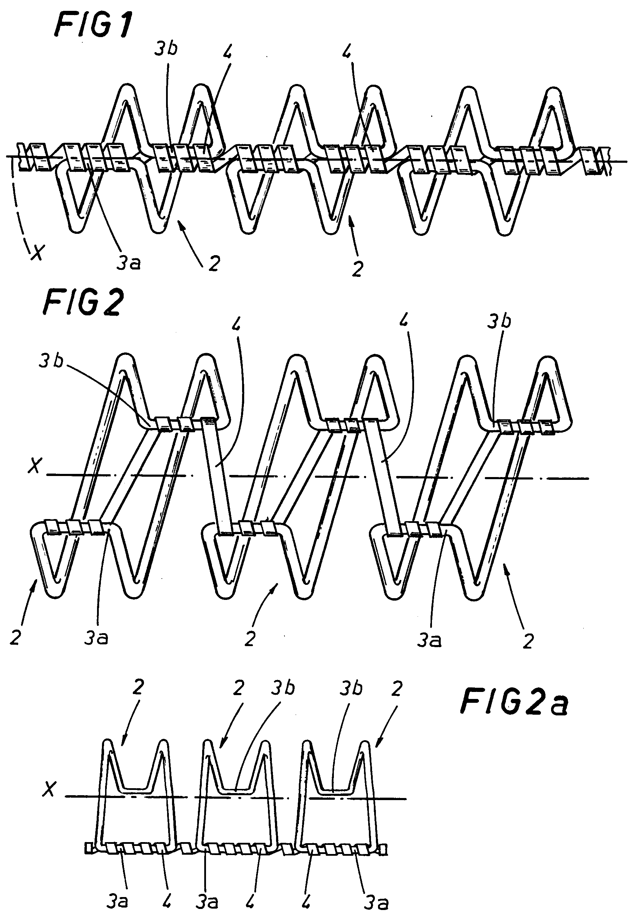

As discernible from the accompanying drawings and with reference in particular to figs 1 and 2, the endoprosthesis to which the invention relates is of the type referred to conventionally in medical circles as a stent, implanted typically in blood vessels, and more especially in coronary arteries; the stent disclosed comprises two elements: a body consisting in a plurality of discrete modules 2 arranged in axial succession one alongside another to form an assembly of open tubular geometry, and a single helicoid element 4 by which the modules 2 are joined along an axis extending parallel with the longitudinal axis X of the assembled stent, and can be made thus to assume a closed configuration.

More exactly, each single module 2 is fashioned in a biocompatible material and exhibits a geometry definable as two "C" members joined one to another at their corresponding free ends with no break in continuity by respective rectilinear and mutually parallel members 3a and 3b. In the example of the drawings, and as will be described further in due course, each module 2, or each pair of "C" members, appears as a portion of a coil with the rectilinear connecting members 3a and 3b offset one from the other in the direction of the longitudinal axis X; in practice, the module is fashioned from a wire of suitable metal, such as stainless steel or an alloy of titanium and nickel, or of platinum and iridium, looped in such a manner that when the stent is in an as yet undeformed position, the two rectilinear members 3a and 3b are staggered, coinciding with a single straight line parallel to the longitudinal axis X.

The helicoid element 4 is fashioned similarly from a biocompatible material (likewise stainless steel, platinum-iridium alloy or titanium-nickel alloy), and consists in a single spiral wound wire that can be coiled around both rectilinear members 3a and 3b of the modules 2 (figs 1 and 2), arranged in linear succession as mentioned above, in such a way as to effect a closure of the modules on a line parallel with the longitudinal axis X, thereby obtaining a single element of enclosed tubular geometry with the modules 2 disposed one alongside another.

In another solution, illustrated in fig 2a, the helicoid element 4 is coiled around only one of the rectilinear members (that denoted 3a): this in the event that radial strength of the stent is not the prime requirement, but rather a greater plastic deformability of the prosthetic structure overall.

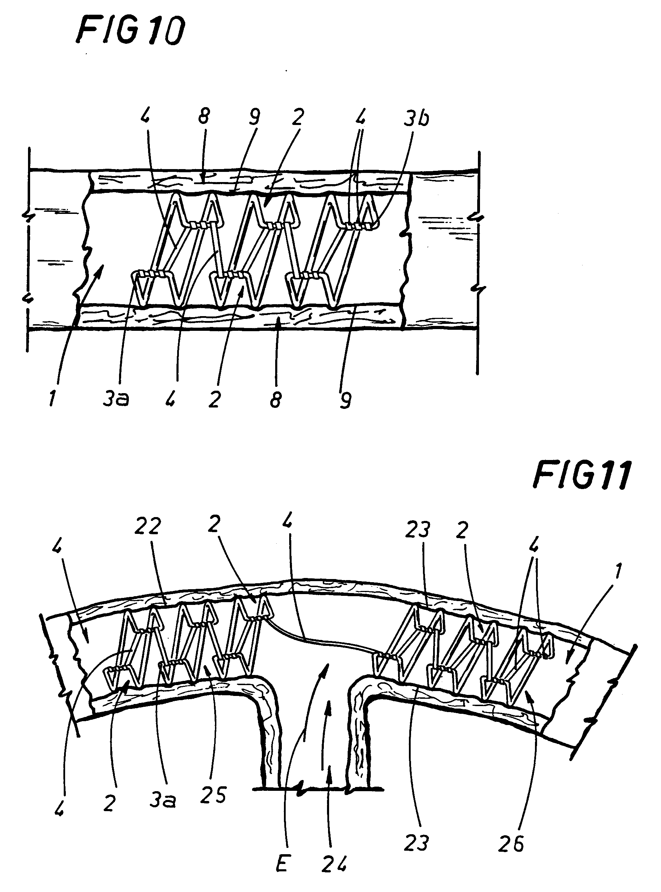

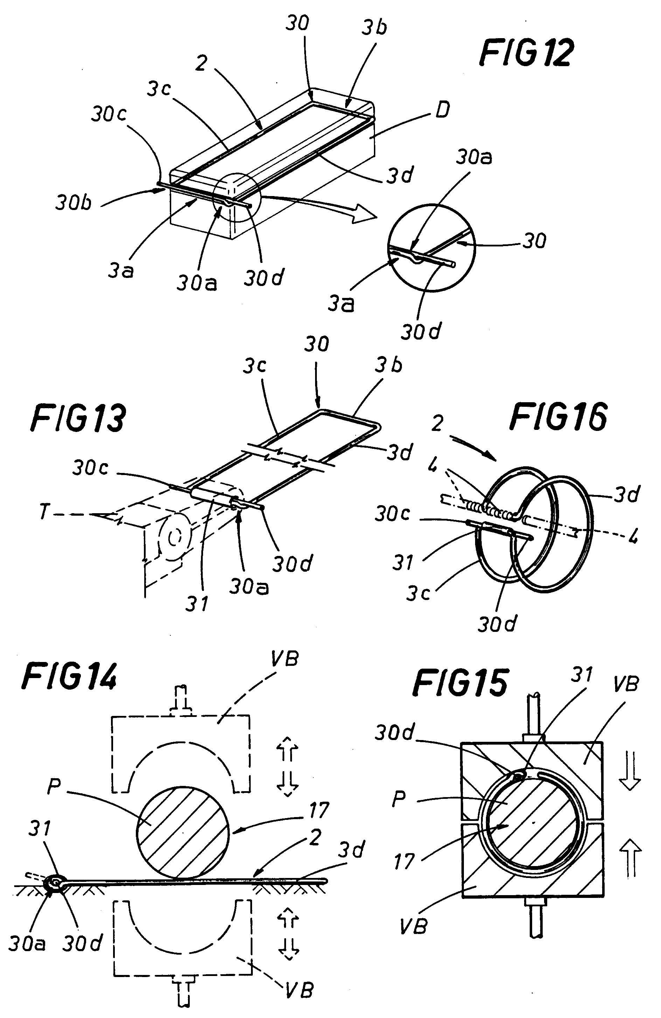

Both the modules 2 and the helicoid element 4 are in fact plastically deformable both in the radial and in the axial direction, so that it is possible to obtain a selective axial distribution of the modules 2 within the blood vessel 1 occupied by the implanted stent (see figs 10 and 11), as well as different configurations of the helicoid element 4.

In a preferred embodiment, the modules 2 or the helicoid element 4 will be carbon coated to prevent the risk of oxidation occurring, over time, as the result of contact between the coils of the spiral and the various component modules. One of the two essential elements, that is, the modules 2 or the helicoid element 4, will be in radiopaque material, i.e. clearly visible when the site of the implant is imaged by external radiography equipment.

Another possible embodiment is illustrated in figs 12 to 19, where the single module 2 consists in a filiform element 30 formed initially with free ends (as will become clear in due course) and presenting a "U" outline of which the terminal portions 30a and 30b coincide with one of the aforesaid parallel members 3a and 3b; the terminal portions 30a and 30b can be secured by a link element 31 (fashioned likewise from a biocompatible material), in such a way that the free ends 30c and 30d of the terminal portions 30a and 30b project from the corresponding ends of the link element 31, affording points such as will anchor positively in the wall of the blood vessel 1. The link element 31 is also embodied in a radiopaque material to allow maximum visibility of the stent when implanted.

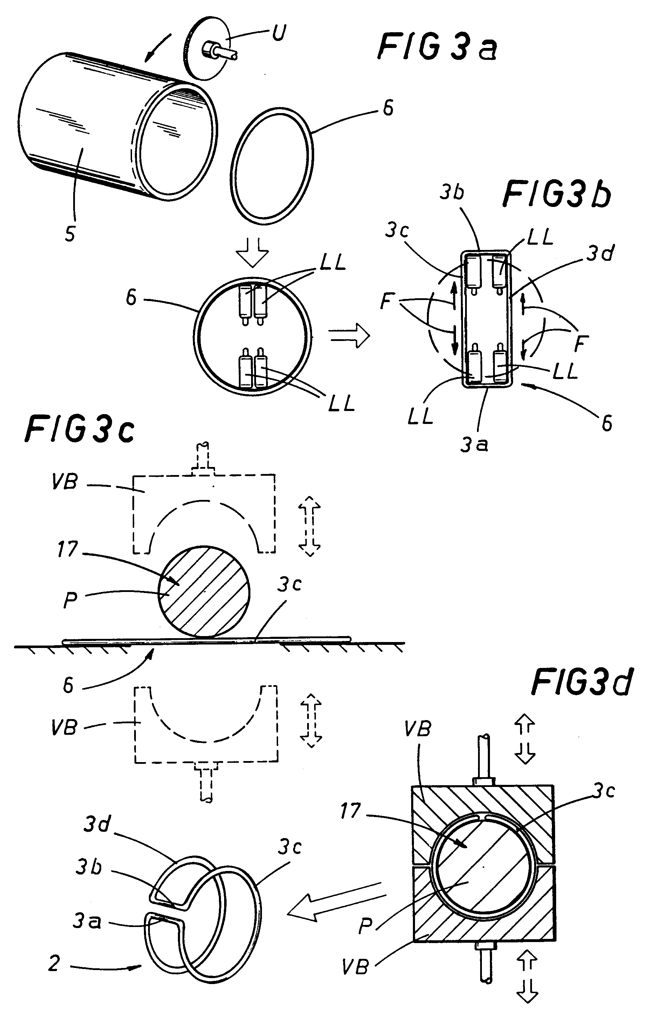

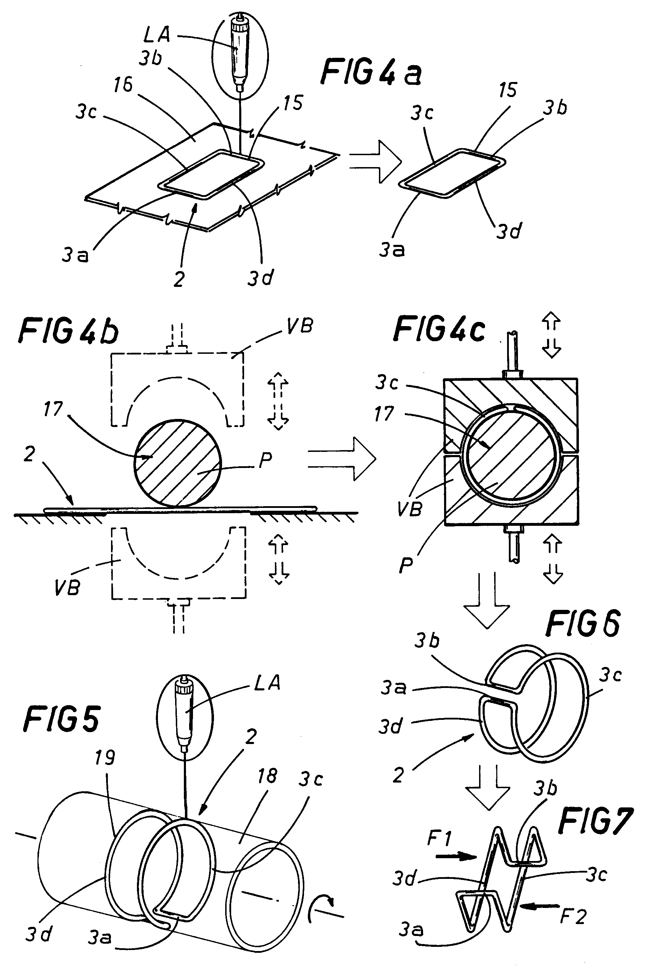

The stent disclosed can be fabricated by a variety of methods: in a first solution, illustrated in figs 3a to 3d, the procedure comprises a first step a) in which a tubular element 5, fashioned from the particular biocompatible material selected for the modules 2, is divided mechanically into a plurality of slender rings 6; given the minute proportions of the modules 2, this will be a precision machining operation (performed for example on a special lathe of conventional type not indicated in the drawings, with a tool U consisting in a precision cutter). In a second step b) illustrated in fig 3b, the single ring 6 is deformed, applying pressure in the radial direction (see arrows denoted F) through the agency of special shaping means LL such that the hitherto circular outline is rendered quadrangular, thereby creating pairs of rectilinear members 3a, 3b, 3c and 3d (where 3a and 3b are the shorter, and 3c and 3d the longer). In a third step c), the individual quadrangular elements obtained from the previous operation are bent (see figs 3c and 3d) utilizing special template means 17 (which comprise a central former P and moving dies VB) in such a manner as to produce a cylindrical or helicoidal geometry (the selection of which will be explained in due course) composed of two nominally circular "C" members 3c and 3d plus the two rectilinear connecting members 3a and 3b already described, which extend parallel and can be either aligned transversely, or offset as in fig 7; thereafter, in a final step d), the helicoid element 4 is coiled around one or both of the two rectilinear connecting members 3a and 3b afforded by the relative plurality of modules 2, as in figs 1, 2, and 2a (the number of modules making up the prosthesis will be determined according to the effective requirement), extending in alignment with a longitudinal axis parallel to the axis X of the stent, so that the configuration ultimately obtained appears tubular when viewed in section.

In a second solution illustrated in figs 4a to 4c, fabrication of the stent commences with the step of cutting an element 15 of the selected biocompatible material from a flat leaf 16 (preferably by means of a laser cutting device LA), in such a way as to obtain a quadrangular shape that will again produce a module 2 exhibiting the two pairs of rectilinear members 3a-3b and 3c-3d; each single quadrangular element 15 is then deformed (see figs 4b and 4c) using template means 17 as described above, in such a way as to convert the geometry of the quadrangle from flat to cylindrical and thus generate a module as shown in fig 6. Finally, the helicoid element 4 is coiled in alignment with a longitudinal axis around one or both of the rectilinear connecting members 3a and 3b of the modules 2 to obtain the tubular configuration (again, as in figs 1 and 2).

In a third solution (indicated in fig 5), modules 2 are created by cutting a thin gauge tube 18 of the selected material (again with a laser device LA) in such a way as to obtain a quadrangular cylindrical element 19 exhibiting two "C" members, which thus will already present the configuration in fig 6; thereafter, the helicoid element 4 is coiled in parallel alignment with the longitudinal axis of the stent around one or both of the two rectilinear connecting members 3a and 3b of the modules 2, in such a way as to obtain the tubular configuration aforementioned.

A fourth solution, illustrated in figs 12 to 16, comprises the initial step of bending a filiform element 30 around a suitable former D (see fig 12) so as to create a module 2 quadrangular in outline, of which the terminal portions 30a and 30b are disposed mutually parallel and adjacent, coinciding with one of the rectilinear connecting members 3a or 3b; the next step is to secure the two terminal portions 30a and 30b, in this instance coinciding with the rectilinear member denoted 3a in fig 13, by means of the link element 31, which is crimped in place using an appropriate tool T. The filiform element 30 is of length such that on completion of this step, the two free ends 30c and 30d, that is to say the extremities of the terminal portions 30a and 30b, will project from the link element 31; thereafter, the filiform element 30 is deformed in such a way as to convert the configuration of the quadrangle from flat to cylindrical, again through the agency of the template means 17 (see figs 14 and 15): this same step results in the creation of two "spurs", afforded by the two free ends 30c and 30d, which serve to anchor the stent positively to the wall of the blood vessel 1 (as described more fully in due course).

Finally, the helicoid element 4 is coiled, parallel with the longitudinal axis X of the stent, around one of the two shorter members 3a and 3b afforded by each module 2 created from a respective filiform element 30, in this instance the member 3b remote from the link element 31 (see fig 16).

In the event that the helicoid element 4 is to be coiled around both of the rectilinear members 3a and 3b (as mentioned previously), the module 2 will be made to assume a helicoid geometry with the two circular "C" members 3c and 3d remaining parallel and the two rectilinear members 3a and 3b mutually offset, aligned along a common horizontal generator (see fig 7, arrows F1 and F2). With the helicoid element 4 engaging both rectilinear members 3a and 3b of the modules, the stent assumes an enclosed tubular configuration affording high resistance to radial pressure (see figs 1 and 2).

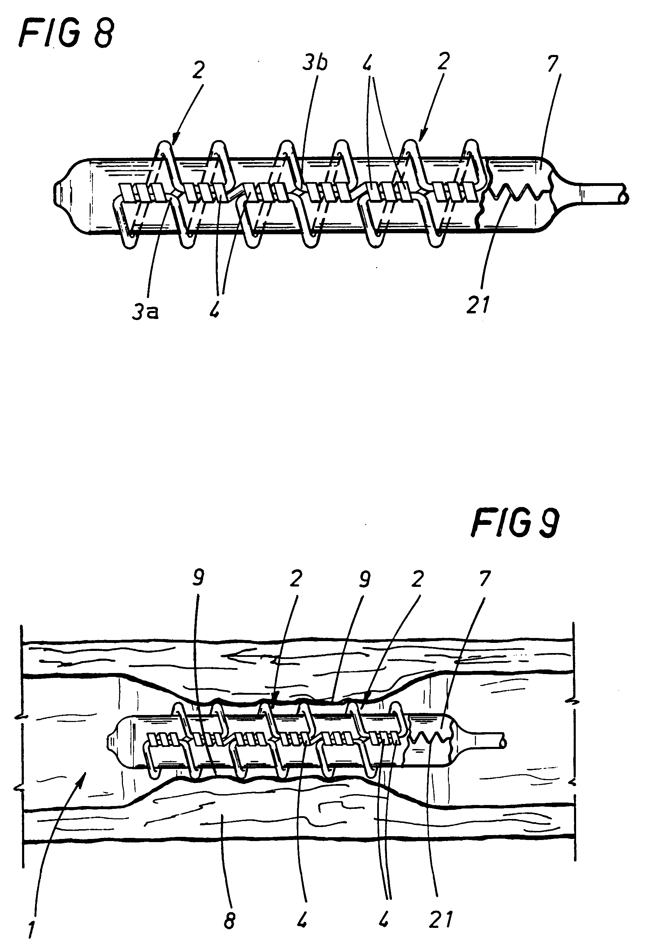

A stent thus described and illustrated is implanted by way of the following simple steps:
- a) the stent is fitted to a catheter 7 such as will allow its insertion into the blood vessel 1 in a contracted configuration, that is to say, with the rectilinear members 3a and 3b of each module 2 parallel or occupying the same plane, the overall length of the prosthesis having been determined preoperatively (see fig 8);
- b) the catheter 7 is inserted transluminally (see fig 9) into the blood vessel 1 and positioned so as to locate the stent at a predetermined site where an occlusion 8 has occurred;
- c) the inserted stent is expanded by means of the catheter 7 to the point of entering into contact with the wall 9 of the blood vessel 1, or rather, deformed plastically in such a manner as to obtain a different radial configuration of the modules 2, and a different axial configuration at least of the helicoid element 4 (see fig 10).

Naturally, the deformation technique will vary according to the type of material from which the stent is fabricated: in effect, use may be made of a conventional balloon catheter (not illustrated) to produce a purely mechanical type of expansion, or alternatively, the stent may be fabricated from a material with thermal shape memory, i.e. treated in such a way that deformation occurs at a given temperature; in this instance the surgeon will use a catheter (shown in figs 8 and 9) incorporating a special heater 21 by which the temperature can be raised to a specified value enabling deformation of the stent.

To advantage, with a stent structured in this way, the surgeon is at liberty, before proceeding with implantation, to engineer the axial configuration of the prosthesis best suited to the pathological condition of the blood vessel, and more especially the measure and distribution of the narrowed parts the implant is intended to restore. As discernible in fig 11, in effect, indicating a situation with two occlusions 22 and 23 affecting the same blood vessel 1, localized on either side of a junction between the vessel in question and a second blood vessel 24, an optimum configuration can be obtained with the stent disclosed by deforming the helicoid element 4 strategically in the axial direction; the effect is to create two discrete portions 25 and 26 distanced one from another, thereby ensuring the patency of the vessel, while leaving the junction between the two adjoining vessels 1 and 24 almost completely open to the flow of blood (indicated by the arrows denoted E).

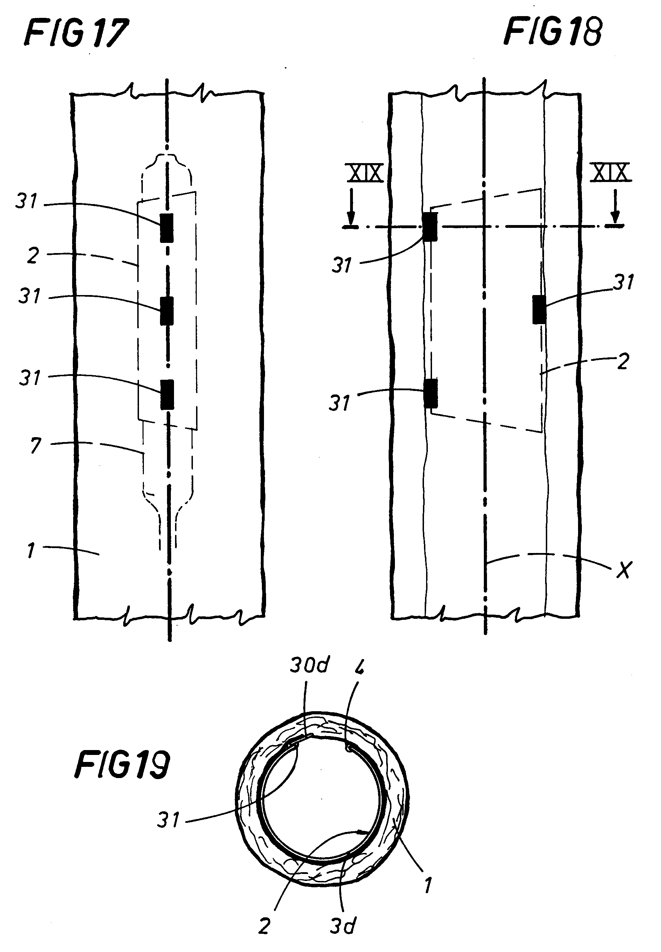

A further advantage is that the positioning of the stent within the blood vessel can be monitored with ease, using radiography, thanks to the radiopacity of the components. This particular feature will be the more evident if use is made of the prosthetic structure incorporating the link element 31, which permits of observing the implant radiographically both during the positioning step (see fig 17), when the elements are aligned along a common axis, and during the subsequent step of expanding the stent (see fig 18), when the selfsame elements 31 should be distributed uniformly on either side of a plane coinciding with the longitudinal axis X, to the end of verifying that the operation is brought to a successful conclusion. Yet another advantage of this solution is that of the anchor spurs afforded by the ends 30c and 30d of the wire (see fig 19), which will penetrate the wall of the vessel 1 when the stent is expanded.

## Claims

1. A coronary endoprosthesis implantable in blood vessels (1),
characterized
in that it comprises:
- a plurality of discrete modules (2) fashioned from a biocompatible material, each exhibiting two "C" shaped members joined one to another at their free ends by respective rectilinear and mutually parallel members (3a, 3b);
- a single helicoid element (4), by way of which the modules (2) are associated one with another in succession, fashioned likewise from a biocompatible material and coiled around at least one of the rectilinear members (3a, 3b) so as to determine substantially a closure of the modules (2) along a direction parallel with a longitudinal axis (X), and in such a way as to establish an integrated prosthetic structure appearing tubular in section with the single modules (2) disposed one alongside another; and,
in that the modules (2) and the helicoid element (4) are plastically deformable both in the radial and in the axial dimension, not least to allow of varying the axial distribution of the modules internally of the blood vessel (1) and obtaining different configurations of the helicoid element.

2. An endoprosthesis as in claim 1, wherein the single helicoid element (4) is coiled around both the rectilinear members (3a, 3b).

3. An endoprosthesis as in claim 1, wherein the single module (2) presents a helicoidal configuration in which the two "C" members are joined by rectilinear connecting members (3a, 3b) offset one from another in the direction of the longitudinal axis (X).

4. An endoprosthesis as in claim 1, wherein the single module (2) consists in a wire of selected metal and the two rectilinear members (3a, 3b) are disposed, at least in an undeformed configuration of the prosthetic structure, offset one from another and aligned on a single straight line parallel to the longitudinal axis (X).

5. An endoprosthesis as in claim 1, wherein at least the modules (2) or the helicoid element (4) will be fashioned from a radiopaque material.

6. An endoprosthesis as in claim 1, wherein the single module (2) is fashioned from stainless steel.

7. An endoprosthesis as in claim 1, wherein the single module (2) is fashioned from an alloy of platinum and iridium.

8. An endoprosthesis as in claim 1, wherein the single module (2) is fashioned from an alloy of titanium and nickel.

9. An endoprosthesis as in claim 1, wherein the helicoid element (4) consists in a spiral wound wire fashioned from stainless steel.

10. An endoprosthesis as in claim 1, wherein the helicoid element (4) consists in a spiral wound wire fashioned from an alloy of platinum and iridium.

11. An endoprosthesis as in claim 1, wherein the helicoid element (4) consists in a spiral wound wire fashioned from an alloy of titanium and nickel.

12. An endoprosthesis as in claim 1, wherein at least the modules (2) or the helicoid element (4) will be carbon coated.

13. An endoprosthesis as in claim 1, wherein the single module (2) consists in a filiform element (30) presenting a quadrangular "U" outline of which the terminal portions (30a, 30b) coincide with one of the parallel members (3a, 3b) and are secured by a link element (31), fashioned from a biocompatible and radiopaque material, in such a way that the free ends (30c, 30d) of the terminal portions (30a, 30b) project from the corresponding ends of the link element (31) as anchor points designed to penetrate the wall of the blood vessel (1).

14. A method of fabricating an endoprosthesis as in claims 1 to 12, comprising the steps of:
- a) dividing and cutting a tubular element (5) into a plurality of slender rings (6), each constituting a single module (2);
- b) deforming each single ring (6) radially in such a way as to change the hitherto circular outline to a substantially quadrangular outline comprising pairs of rectilinear members (3a, 3b, 3c, 3d);
- c) bending each single module (2) of quadrangular outline to a cylindrical shape through the agency of template means (17) in such a way as to obtain two mutually parallel and substantially circular members (3c, 3d) of "C" profile;
- d) coiling the helicoid element (4) in alignment with a longitudinal axis around at least one of the rectilinear members (3a, 3b) afforded by each of the single modules (2) making up the prosthetic structure, in such a way as to obtain a tubular configuration of the structure.

15. A method of fabricating an endoprosthesis as in claims 1 to 12, comprising the steps of:
- a) cutting an element (15) from a flat leaf (16) of material so as to produce a quadrangular outline comprising pairs of rectilinear members (3a, 3b, 3c, 3d);
- b) bending each single quadrangular element (15) from a flat profile to a substantially cylindrical profile through the agency of template means (17);
- c) coiling the helicoid element (4) in alignment with a longitudinal axis around at least one of the rectilinear members (3a, 3b) afforded by each of the single modules (2) making up the prosthetic structure, in such a way as to obtain a tubular configuration of the structure.

16. A method of fabricating an endoprosthesis as in claims 1 to 12, comprising the steps of:
- a) cutting a thin gauge tubular element (18) so as to obtain an element (19) of quadrangular and substantially cylindrical geometry exhibiting two "C" members and constituting the single module (2);
- b) coiling the helicoid element (4) in alignment with a longitudinal axis around at least one of the rectilinear members (3a, 3b) afforded by each of the single modules (2) making up the prosthetic structure, in such a way as to obtain a tubular configuration of the structure.

17. A method of fabricating an endoprosthesis as in claim 13, comprising the steps of:
- a) bending a filiform element (30) in such a way as to create a module (2) quadrangular in outline, of which the two terminal portions (30a, 30b) are disposed mutually parallel and adjacent, coinciding with one of the rectilinear members (3a, 3b) of the module (2);
- b) securing the rectilinear member (3a, 3b) that coincides with the terminal portions (30a, 30b), by means of the link element (31), such that the free ends (30c, 30d) afforded by the extremities of the terminal portions (30a, 30b) project from opposite ends of the link element (31);
- c) bending each single quadrangular filiform element (30) from a flat profile to a substantially cylindrical profile through the agency of template means (17);
- d) coiling the helicoid element (4) in alignment with a longitudinal axis around at least one of the rectilinear members (3a, 3b) afforded by each of the single modules (2) formed from corresponding filiform elements (30) and making up the prosthetic structure, in such a way as to obtain a tubular configuration of the structure.

18. A method as in claim 14 or 15 or 16, wherein the helicoid element (4) is coiled around both of the rectilinear members (3a, 3b) in such a way that each module (2) exhibits an enclosed tubular configuration when seen in section.

19. A method as in claim 14 or 15 or 16, wherein the step of coiling the helicoid element (4) around at least one of the rectilinear members (3a, 3b) is preceded by a bending step during which the module (2) assumes a helicoidal configuration comprising two mutually parallel and essentially circular members (3c, 3d) of "C" profile interconnected by two rectilinear members (3a, 3b) disposed mutually offset and aligned on a common longitudinal axis.

## Patentansprüche

1. Koronare Endoprothese, einsetzbar in Blutgefässe (1), **dadurch gekennzeichnet**, dass sie wie folgt enthält:
- eine Mehrzahl von unabhängigen Gliedern (2), hergestellt aus einem körperverträglichen Material, von denen jedes zwei C-förmig ausgebildete Teile aufweist, die an ihren freien Enden durch jeweilige geradlinige und parallel zueinander verlaufende Abschnitte (3a, 3b) miteinander verbunden sind;
- ein einzelnes, schneckenförmiges Element (4), durch welches die Glieder (2) aufeinanderfolgend eines mit dem anderen verbunden sind, und das ebenfalls aus einem körperverträglichen Material hergestellt und um wenigstens eins der geradlinigen Abschnitte (3a, 3b) gewunden ist, so dass im wesentlichen ein Verschliessen der Glieder (2) entlang einer Richtung parallel zu einer Längsachse (X) beschrieben wird, und zwar auf solche Weise, dass sich eine integrierte Prothesenstruktur ergibt, die im Schnitt rohrförmig erscheint mit den einzelnen Gliedern (2) der Länge nach eines nach dem anderen angeordnet; und **dadurch**, dass die Glieder (2) und das schneckenförmige Element (4) sei es in der radialen wie auch in der axialen Ausdehnung plastisch verformbar sind, nicht zuletzt darum, dass eine Veränderung der axialen Verteilung der Glieder im Inneren des Blutgefässes (1) erlaubt ist und unterschiedliche Darstellungen des schneckenförmigen Elementes erhalten werden können.

2. Endoprothese nach Patentanspruch 1, bei welcher das einzelne schneckenförmige Element (4) um beide geradlinigen Abschnitte (3a, 3b) gewunden ist.

3. Endoprothese nach Patentanspruch 1, bei welcher das einzelne Glied (2) eine schneckenförmige Darstellung aufweist, in welcher die beiden C-förmigen Teile durch geradlinige Verbindungsabschnitte (3a, 3b) miteinander verbunden sind, welche in Richtung der Längsachse (X) einer zu dem anderen versetzt sind.

4. Endoprothese nach Patentanspruch 1, bei welcher die einzelnen Glieder (2) aus einem ausgewählten Metalldraht bestehen und die beiden geradlinigen Abschnitte (3a, 3b) wenigstens in einem nicht verformten Bereich der Prothesenstruktur einer zu dem anderen versetzt angeordnet und in einer einzigen geraden Linie parallel zu der Längsachse (X) ausgerichtet sind.

5. Endoprothese nach Patentanspruch 1, bei welcher wenigstens die Glieder (2) oder das schneckenförmige Element (4) aus einem strahlenundurchlässigen Material hergestellt ist.

6. Endoprothese nach Patentanspruch 1, bei welcher das einzelne Glied (2) aus rostfreiem Stahl hergestellt ist.

7. Endoprothese nach Patentanspruch 1, bei welcher das einzelne Glied (2) aus einer Legierung aus Platin und Iridium hergestellt ist.

8. Endoprothese nach Patentanspruch 1, bei welcher das einzelne Glied (2) aus einer Legierung aus Titan und Nickel hergestellt ist.

9. Endoprothese nach Patentanspruch 1, bei welcher das schneckenförmige Element (4) aus einem spiralförmig gewundenen Draht besteht, der aus rostfreiem Stahl hergestellt ist.

10. Endoprothese nach Patentanspruch 1, bei welcher das schneckenförmige Element (4) aus einem spiralförmig gewundenen Draht besteht, der aus einer Legierung aus Platin und Iridium hergestellt ist.

11. Endoprothese nach Patentanspruch 1, bei welcher das schneckenförmige Element (4) aus einem spiralförmig gewundenen Draht besteht, der aus einer Legierung von Titan und Nickel hergestellt ist.

12. Endoprothese nach Patentanspruch 1, bei welcher wenigstens die Glieder (2) oder das schneckenförmige Element (4) mit Kohlenstoff überzogen sind.

13. Endoprothese nach Patentanspruch 1, bei welcher das einzelne Glied (2) aus einem fadenförmigen Element (30) besteht, welches eine viereckige U-förmige Ausbildung aufweist, deren Endabschnitte (30a, 30b) mit einem der parallelen Abschnitt (3a, 3b) übereinstimmen und durch ein Bindeelement (31), hergestellt aus körperverträglichem und strahlenundurchlässigem Material, befestigt sind, und zwar auf solche Weise, dass die freien Enden (30c, 30d) der Endabschnitte (30a, 30b) aus den entsprechenden Enden des Bindeelementes (31) als Verankerungspunkte herausragen, dazu bestimmt, in die Wand des Blutgefässes (1) einzudringen.

14. Verfahren zur Herstellung einer Endoprothese nach den Patentansprüchen von 1 bis 12, enthaltend folgende Schritte:
- a) Trennen und Schneiden eines rohrförmigen Elementes (5) in eine Mehrzahl von dünnen Ringen (6), jeder davon ein einzelnes Glied (2) bildend;
- b) Verformen eines jeden einzelnen Ringes (6) in radialer Richtung auf solche Weise, dass dessen bisher kreisförmige Ausbildung eine im wesentlichen viereckige Form erhält, einschliesslich von Paaren geradliniger Abschnitte (3a, 3b, 3c, 3d);
- c) Biegen eines jeden einzelnen Gliedes (2) von einer viereckigen Form zu einer zylindrischen Form mit Hilfe von Schablonen (17) auf solche Weise, das zwei zueinander parallel verlaufende und im wesentlichen kreisförmige Abschnitte (3c, 3d) von einem C-Profil erhalten werden;
- Wickeln des schneckenförmigen Elementes (4) in Ausrichtung zu einer Längsachse um wenigstens einen der geradlinigen Abschnitte (3a, 3b), die von jedem der die Prothesenstruktur bildenden einzelnen Glied (2) aufgewiesen sind, um somit eine rohrförmige Darstellung der Struktur zu erhalten.

15. Verfahren zur Herstellung einer Endoprothese nach den Patentansprüchen von 1 bis 12, enthaltend folgende Schritte:
- a) Schneiden eines Elementes (15) aus einer flachen Folie (16) von Material, so dass eine viereckige Form einschliesslich Paaren von geradlinigen Abschnitten (3a, 3b, 3c, 3d) erhalten wird;
- b) Biegen eines jeden einzelnen viereckigen Elementes (15) aus einem flachen Profil zu einem im wesentlichen zylindrischen Profil mit Hilfe von Schablonen (17);
- c) Wickeln des schneckenförmigen Elementes (4) in Ausrichtung zu einer Längsachse um wenigstens einen der geradlinigen Abschnitte (3a, 3b), die von jedem der die Prothesenstruktur bildenden einzelnen Glied (2) aufgewiesen sind, um somit eine rohrförmige Darstellung der Struktur zu erhalten.

16. Verfahren zur Herstellung einer Endoprothese nach den Patentansprüchen von 1 bis 12, enthaltend folgende Schritte:
- a) Schneiden eines dünnwandigen rohrförmigen Elementes (18), um ein Element (19) von viereckiger und im wesentlichen zylindrischer Geometrie zu erhalten, welches die Form von zwei "C" hat und das einzelne Glied (2) bildet;
- b) Wickeln des schneckenförmigen Elementes (4) in Ausrichtung zu einer Längsachse um wenigstens einen der geradlinigen Abschnitte (3a, 3b), die von jedem der die Prothesenstruktur bildenden einzelnen Glied (2) aufgewiesen sind, um somit eine rohrförmige Darstellung der Struktur zu erhalten.

17. Verfahren zur Herstellung eines Endprothese nach Patentanspruch 13, enthaltend die folgenden Schritte:
- a) Biegen eines fadenförmigen Elementes (30) auf solch eine Weise, dass ein in seiner Form viereckiges Glied (2) gebildet wird, dessen zwei Endabschnitte (30a, 30b) zueinander parallel und angrenzend angeordnet sind, wobei sie mit einem der geradlinigen Abschnitte (3a, 3b) des Gliedes (2) übereinstimmen;
- b) Befestigung des geradlinigen Abschnittes (3a, 3b), der mit den Endabschnitten (30a, 30b) übereinstimmt, mit Hilfe eines Bindeelementes (31), so dass die freien Enden (30c, 30d), die von den Enden der Endabschnitte (30a, 30b) aufgewiesen werden, aus den entgegengesetzten Enden des Bindeelementes (31) herausragen;
- c) Biegen eines jeden einzelnen, viereckigen, fadenförmigen Elementes (30) aus einem flachen Profil zu einem im wesentlichen zylindrischen Profil mit Hilfe einer Schablone (17);
- d) Wickeln des schneckenförmigen Elementes (4) in Ausrichtung zu einer Längsachse um wenigstens einen der geradlinigen Abschnitte (3a, 3b), die von jedem aus den entsprechenden fadenförmigen Elementen (30) geformten und die Prothesenstruktur bildenden einzelnen Glied (2) aufgewiesen sind, um somit eine rohrförmige Darstellung der Struktur zu erhalten.

18. Verfahren nach Patentanspruch 14 oder 15 oder 16, bei welchem das schneckenförmige Element (4) um beide geradlinige Abschnitte (3a, 3b) gewickelt wird, und zwar auf solche Weise, dass jedes Glied (2) im Schnitt gesehen eine geschlossene rohrförmige Darstellung aufweist.

19. Verfahren nach Patentanspruch 14 oder 15 oder 16, bei welchem der Wickelphase des schneckenförmigen Elementes (4) um wenigstens einen der geradlinigen Abschnitte (3a, 3b) eine Biegephase vorausgeht, während der das Glied (2) eine schneckenförmige Darstellung annimmt, einschliesslich von zwei zueinander parallelen und im wesentlichen kreisförmigen Abschnitten (3c, 3d) von einem "C-Profil, miteinander verbunden durch zwei geradlinige Abschnitte (3a, 3b), die versetzt zueinander und entlang einer gemeinsamen Längsachse angeordnet sind.

## Revendications

1. Endoprothèse coronaire pouvant être implantée dans des vaisseaux sanguins (1),
caractérisée en ce qu'elle comprend:
- une pluralité de modules (2) indépendants réalisés dans un matériau biocompatible, présentant chacun deux éléments en forme de "C" reliés l'un à l'autre au niveau de leurs extrémités libres par des segments rectilignes respectifs (3a, 3b) réciproquement parallèles:
- un unique élément hélicoïdal (4), par l'intermédiaire duquel les modules (2) sont associés l'un avec l'autre en succession, également réalisé dans un matériau biocompatible et enroulé autour d'au moins un des segments rectilignes (3a, 3b) de façon à fermer en substance les modules (2) suivant une direction parallèle à un axe longitudinal (X), et de façon à créer une structure prosthétique intégrée présentant une section tubulaire avec les modules (2) unitaires placés l'un à côté de l'autre; et,
en ce que les modules (2) et l'élément hélicoïdal (4) sont plastiquement déformables tant en direction radiale qu'en direction axiale et pour pouvoir faire varier la distribution axiale des modules à l'intérieur du vaisseau sanguin (1) et obtenir différentes configurations de l'élément hélicoïdal.

2. Endoprothèse selon la revendication 1, caractérisée en ce que l'élément hélicoïdal (4) est enroulé autour des deux segments rectilignes (3a, 3b).

3. Endoprothèse selon la revendication 1, caractérisée en ce que chaque module (2) présente une configuration hélicoïdale dans laquelle les deux éléments en forme de "C" sont reliés par des segments de liaison rectilignes (3a, 3b) décalés l'un de l'autre suivant la direction de l'axe longitudinal (X).

4. Endoprothèse selon la revendication 1, caractérisée en ce que chaque module (2) consiste en un fil de métal sélectionné et les deux segments rectilignes (3a, 3b) sont placés, au moins dans une configuration non déformée de la structure prosthétique, décalés l'un de l'autre et alignés sur une unique droite parallèle à l'axe longitudinal (X).

5. Endoprothèse selon la revendication 1, caractérisée en ce qu'au moins les modules (2) ou l'élément hélicoïdal (4) seront réalisés dans un matériau radiopaque.

6. Endoprothèse selon la revendication 1, caractérisée en ce que chaque module (2) est réalisé en acier inoxydable.

7. Endoprothèse selon la revendication 1, caractérisée en ce que chaque module (2) est réalisé dans un alliage de platine et iridium.

8. Endoprothèse selon la revendication 1, caractérisée en ce que chaque module (2) est réalisé dans un alliage de titane et nickel.

9. Endoprothèse selon la revendication 1, caractérisée en ce que l'élément hélicoïdal (4) consiste en un fil spiralé réalisé en acier inoxydable.

10. Endoprothèse selon la revendication 1, caractérisée en ce que l'élément hélicoïdal (4) consiste en un fil spiralé réalisé dans un alliage de platine et iridium.

11. Endoprothèse selon la revendication 1, caractérisée en ce que l'élément hélicoïdal (4) consiste en un fil spiralé réalisé dans un alliage de titane et nickel.

12. Endoprothèse selon la revendication 1, caractérisée en ce que au moins les modules (2) ou l'élément hélicoïdal (4) seront revêtus de carbone.

13. Endoprothèse selon la revendication 1, caractérisée en ce que chaque module (2) consiste en un élément filiforme (30) présentant une conformation quadrangulaire en "U" dont les portions terminales (30a, 30b) coïncident avec l'un des segments parallèles (3a, 3b) et sont fixées par un élément de liaison (31), réalisé dans un matériau biocompatible et radiopaque, de sorte que les extrémités libres (30c, 30d) des portions terminales (30a, 30b) doivent saillir des extrémités correspondantes de l'élément de liaison (31), définissant ainsi des points d'ancrage dans la paroi du vaisseau sanguin (1).

14. Méthode de fabrication d'une endoprothèse selon les revendications de 1 à 12, comprenant les phases suivantes:
- a) division et coupe d'un élément tubulaire (5) en une pluralité d'anneaux (6) d'épaisseur réduite, constituant chacun un module (2);
- b) déformation radiale de chaque anneau (6) de façon à passer de la configuration circulaire à une configuration en substance quadrangulaire comprenant des paires de segments rectilignes (3a, 3b, 3c, 3d);
- c) pliage de chacun des modules (2) quadrangulaires pour leur donner une forme cylindrique au moyen d'un gabarit (17) de façon à obtenir deux éléments réciproquement parallèles et en substance circulaires (3c, 3d) présentant un profil en "C";
- d) enroulement de l'élément hélicoïdal (4) suivant un axe longitudinal autour d'au moins l'un des segments rectilignes (3a, 3b) présentés par chacun des modules (2) définissant la structure prosthétique, de manière à donner à la structure une configuration tubulaire.

15. Méthode de fabrication d'une endoprothèse selon les revendications de 1 à 12, comprenant les phases suivantes:
- a) découpe d'un élément (15) à partir d'une feuille plate (16) de matériau de façon à obtenir une configuration quadrangulaire comprenant des paires de segments rectilignes (3a, 3b, 3c, 3d);
- b) pliage de chaque élément quadrangulaire (15) pour passer d'une conformation plate à une conformation en substance cylindrique au moyen d'un gabarit (17);
- c) enroulement de l'élément hélicoïdal (4) suivant un axe longitudinal autour d'au moins l'un des segments rectilignes (3a, 3b) présentés par chacun des modules (2) définissant la structure prosthétique, de manière à donner à la structure une configuration tubulaire.

16. Méthode de fabrication d'une endoprothèse selon les revendications de 1 à 12, comprenant les phases suivantes:
- a) découpe d'un élément tubulaire (18) d'épaisseur réduite pour obtenir un élément (19) quadrangulaire et en substance cylindrique présentant deux éléments en "C" et constituant le module (2) unitaire;
- b) enroulement de l'élément hélicoïdal (4) suivant un axe longitudinal autour d'au moins l'un des segments rectilignes (3a, 3b) présentés par chacun des modules (2) définissant la structure prosthétique, de manière à donner à la structure une configuration tubulaire.

17. Méthode de fabrication d'une endoprothèse selon la revendication 13, comprenant les phases suivantes:
- a) déformation d'un élément filiforme (30) de façon à créer un module (2) quadrangulaire, dont les deux portions terminales (30a, 30b) sont réciproquement parallèles et adjacentes, coïncidant avec l'un des segments rectilignes (3a, 3b) du module (2);
- b) fixation du segment rectiligne (3a, 3b) qui coïncide avec les portions terminales (30a, 30b), au moyen de l'élément de liaison (31), de façon à ce que les extrémités libres (30c, 30d) présentées par les extrémités des portions terminales (30a, 30b) saillent des extrémités opposées de l'élément de liaison (31);
- c) pliage de chaque élément filiforme quadrangulaire (30) pour passer d'une conformation plate à une conformation en substance cylindrique au moyen d'un gabarit (17);
- d) enroulement de l'élément hélicoïdal (4) suivant un axe longitudinal autour d'au moins l'un des segments rectilignes (3a, 3b) présentés par chacun des modules (2) formés par des éléments filiformes (30) correspondants et définissant la structure prosthétique, de manière à donner à la structure une configuration tubulaire.

18. Méthode selon la revendication 14 ou 15 ou 16, caractérisée en ce que l'élément hélicoïdal (4) est enroulé autour des deux segments rectilignes (3a, 3b) de manière à ce que chaque module (2) présente une configuration tubulaire fermée quand vue en coupe.

19. Méthode selon la revendication 14 ou 15 ou 16, caractérisée en ce que la phase d'enroulement de l'élément hélicoïdal (4) autour d'au moins l'un des segments rectilignes (3a, 3b) est précédée d'une phase de déformation pendant laquelle le module (2) prend une configuration hélicoïdale comprenant deux éléments réciproquement parallèles et en substance circulaires (3c, 3d) en forme de "C" reliés entre eux par deux segments rectilignes (3a, 3b) réciproquement décalés et alignés sur un axe longitudinal commun.
